# EUROPEAN PATENT SPECIFICATION

(11) **EP 3 382 030 B1**
(45) Date of publication and mention of the grant of the patent: **07.06.2023**
(21) Application number: 17164444.6
(22) Date of filing: 31.03.2017
(51) Int. Cl.: C12P 3/00, C12P 7/24, C12M 1/00, C12M 1/107, C05F 11/00

(54) **METHOD AND INSTALLATION FOR BIOGAS AND HYDROGEN PRODUCTION, AND FERTILIZERS CONTAINING CHELATES OBTAINED BY THIS METHOD**
VERFAHREN UND ANLAGE ZUR HESTELLUNG VON BIOGAS UND WASSERSTOFF, UND DÜNGEMITTEL, WELCHE DIE MIT DIESEM VERFAHREN ERHALTENEN CHELATE ENTHALTEN.
PROCÉDÉ ET INSTALLATION POUR LA PRODUCTION DE BIOGAZ ET D'HYDROGÈNE, ET ENGRAIS CONTENANT DE CHÉLATES OBTENUS PAR CE PROCÉDÉ

(43) Date of publication of application: 03.10.2018
(73) Proprietor: Ignaciuk, Henryk, 21-560 Miedzyrzec Podlaski (PL); Ignaciuk, Jakub, 21-560 Miedzyrzec Podlaski (PL)
(72) Inventor: Ignaciuk, Henryk, 21-560 Miedzyrzec Podlaski (PL); Ignaciuk, Jakub, 21-560 Miedzyrzec Podlaski (PL)
(74) Representative: Kalita, Lucjan

(56) References cited:
- EP-A1- 2 799 534
- US-A1- 2010 032 370
- US-B1- 8 741 142
- SIEDLECKA: "Report from the 23rd congress of the Polish association of agricultural and agribusiness economists "Bioeconomy in rural development and agribusiness", Biala Podlaska, September 7-9, 2016", ECREG STUDIES (ECONOMIC AND REGIONAL STUDIES), vol. 9, 2016, pages 143-149, XP002771115,
- BUDZIANOWSKI: "A review of potential innovations for production, conditioning and utilization of biogas with multiple-criteria assessment", RENEWABLE AND SUSTAINABLE ENERGY REVIEWS, vol. 54, 11 November 2015 (2015-11-11), pages 1148-1171, XP029326341,
- MIR ET AL: "Design considerations and operational performance of anaerobic digester: A review", COGENT ENGINEERING, vol. 3, 2016, pages 1-20, XP002771121,
- STRÄUBER ET AL: "Metabolic and microbial community dynamics during the anaerobic digestion of maize silage in a two-phase process", APPLIED MICROBIOLOGY AND BIOTECHNOLOGY, vol. 100, 2016, pages 479-491, XP035870363,
- KIM ET AL: "Effects of temperature and hydraulic retention time on anaerobic digestion of food waste", JOURNAL OF BIOSCIENCE AND BIOENGINEERING, vol. 102, 2006, pages 328-332, XP028042221,
- GAMA ET AL: "Optimisation of enzymatic hydrolysis of apple pomace for production of biofuel and refinery chemicals using commercial enzymes", 3 BIOTECH, 2015, pages 1-13, XP002771122, DOI: 10.1007/s13205-015-0312-7
- IGNACIUK: "Proces fermentacji w biogazowniach. Nowa ustawa. Oplacalnosc biogazowni", Lubelskie Tagi Energetyczne (ENERGETICS), November 2017 Seminarium paper, 2017, pages 1-2, XP002771116, Retrieved from the Internet: URL:http://www.energetics.targi.lublin.pl/ pl/56/czwartek_20_11 [retrieved on 2017-06-14]

## Description

The object of the invention is a method how to conduct the hydrogen-methane fermentation process, which results in high efficiency of biogas and hydrogen. Moreover, the invention object includes also vegetable fertilizer obtained as a liquid digestate.

Commonly known methods of biogas production are based on fermentation technology of agricultural biomass and waste without oxygen access, in the range of psychrophilic (18-28°C), mesophilic (32-43°C) and thermophilic temperatures (52-68°C).

In China, there are predominantly small, oval fermenters with capacity between several up to several tens of cubic meters, with fermentation of various types of waste produced by small farms such as animal feces, waste biomass, etc., mostly in psychrophilic technology, i.e. without heating. Such fermentation is usually carried out in individual fermentation tanks, where pH value is close to neutral and amounts 7.1 to 7.5.

Successively, in Europe, biogas industry is dominated by NaWaRo German technology or related ones. These are installations consisting of circular concrete tanks mostly with diameter 24 to 36 m, covered with a flexible coating, which produces biogas as a result of methane fermentation of the substrates. The process is running at overpressure between 3 and 5 mbar. The substrates are primarily silages, mainly from corn but also from cereals and animal feces: manure, slurry and manure, wherein the substrates of one kind are implemented and their possible change is introduced cautiously and slowly for about 20 days.

Under Polish conditions related to lower profitability of biogas production compared to Germany, the substrates used in NaWaRo technology are supplemented with small amounts of waste from agri-food processing such as distillery, vegetable waste, fruit pulp, etc. The basic principle in existing fermentation technologies is to maintain the pH with value at least 7, since from the level 6.8 the metanogenic inhibition occurs and methane production stops.

There are known the methods of pretreatment of the substrates for fermentation in the process of isolated hydrolysis performed in initial tank. According to data provided by the DBFZ (Deutsche Biomasseforschungszentrum) in Leipzig, Germany, which monitors the biogas market commissioned by the Federal Ministry of Agriculture, Germany has several hundred biogas plants equipped with additional hydrolysis tank, and pH of the hydrolysis process ranges between 6.2 and 5 8 and most often at 5.8-6.2. Furthermore, gases that are a hydrolysis product, that is primarily CO₂ and H₂, with H₂S admixture and NH₃, are brought out through the filters reducing unpleasant odors.

It should be highlighted, that exit of CO₂ and especially H₂ outside the biogas plant, reduces the efficiency of fermentation, because both hydrogen and carbon dioxide are methane building blocks formed by methane bacteria at the final stage of the fermentation process. According to DBFZ data, in Germany and anywhere in the world, no hydrolysers working below pH = 5 have been found, and obtaining such a low pH in industrial installations is considered to be impossible and unfavorable for the process.

There are a number of commercially viable hydrogen production technologies operating on an industrial scale. However, they are based on physicochemical or electrolytic processes rather than on biological ones.

Production of hydrogen from biomass or bio-waste processing is subject to numerous studies, but so far performed on a laboratory scale. The scientific leaders are Japan and the European Union. In Japan, production of bio-hydrogen has been developed by the Hydrogen Innovation Town Business Research Group under Blue Tower technology. However, taking into account the production method of hydrogen, it should be marked that this technology is not related to biological processes because it is based on pyrolysis and combustion processes. Whereas, the concept of bio-hydrogen is used only because the base substrate used for pyrolysis process is biowaste - a dried sludge. The technology scheme is available at http://biomassmagazine.com/articles/8088/japanese-group-plans-to-commercialize-biohydrogen-production

In turn, in European Union, the project that aiming to develop hydrogen production using microbial activity was the HYVOLUTION (integrated project 019825-SES6) implemented in 2006-10. This project has developed the production of bio-hydrogen from biomass under the HYVOLUTION technology consisting of treating the plant substrate with a strong alkaline compound in order to accelerate cellulose compounds degradation, followed by pre-mesophilic fermentation, then thermophilic fermentation, requiring heating up to 70°C in photo bioreactors. As a consequence, the cost of hydrogen production, using developed set of photo bioreactors, was very high and thus rather excluded large-scale industrial use of this technology.

The publication by STRÄUBER et al, "Metabolic and microbial community dynamics during the anaerobic digestion of maize silage in a two-phase process", APPLIED MICROBIOLOGY AND BIOTECHNOLOGY, (2016), vol. 100, discloses a two-phasic anaerobic digestion processes, which can be used for biogas production on demand or a combined chemicals/bioenergy production. In this method hydrolysis/acidogenesis is separated from acetogenesis/methanogenesis. The process is conducted accordingly: the first hydrolytic/acidogenic stage in a leach bed reactor with percolation consisted of four glass columns, and the second acetogenic/methanogenic stage in a hybrid glass reactor. The temperature of the process was 37°C in both phases.

The publication by KIM et al, "Effects of temperature and hydraulic retention time on anaerobic digestion of food waste", JOURNAL OF BIOSCIENCE AND BIOENGINEERING, (2006), vol. 102, reveals a modified three-stage methane fermentation system developed to digest food waste efficiently. This system is consisted of three stages: semianaerobic hydrolysis, anaerobic acidogenesis and strictly anaerobic methanogenesis. Operation temperature was adjusted from 30°C to 55°C, and the hydraulic retention time ranged from 8 to 12 days.

It is known from the patent publication US8741142 (B1) four stages fermentation process conducted in a multistage anaerobic digester for treatment of wastewater including complex organic polymers. The process includes a hydrolysis stage converting the complex organic polymers received at an inlet to solubilized monomers at an outlet; an acidogenesis stage, coupled to the outlet of the hydrolysis stage, converting the solubilized monomers into intermediate products at an outlet of the acidogenesis stage; an acetogenesis stage, coupled to the outlet of the acidogenesis stage, converting the intermediate products into simple molecules at an outlet of the acetogenesis stage; and a methanogenesis stage, coupled to the outlet of the acidogenesis stage, converting the simple molecules to an end product. The end product including a quantity of methane and a quantity of carbon dioxide.

From publication of patent description EP0806404 (A2), a method of treating of household waste or similar substances is known, and comprises the following stages: (A) fragmentation the fraction in a pre-treatment stage, for example crushing; (B) removal of inert substances; (C) conducting an anaerobic process during which metals are released from chelating compound in the hydrolysis and acid fermentation stages to convert them into ionized water-soluble form; (D) the phase containing dissolved metals is separated from the precipitate; (E) the sludge is delivered to the methane manufacturing plant; (F) decantation to separate the fermentation sludge from the residue and (g) the fermentation sludge is dried using an external heat source and utilized as a solid fertilizer bed with dry matter content higher than 55%. The method thus shows the preparation of the fertilizer in the typical stage of hydrolysis process at pH 5.5-6.5 and in the specific fermentation stage.

The patent application US2010032370A1 discloses relates to anaerobic digester construction. Further it discloses a method for biogas (methane) production with the use of digester. The described method relates to a classical method for methan production with the use of methanogenic bacteria inoculum. The C:N ratio of the substrates is maintained in the range from about 15:1 to about 25:1, and preferably from about 20:1 to 25:1.

The aim of the invention is to propose highly efficient methods for biogas and hydrogen production, with high degree of marge of substrate formation.

This object is accomplished by the methods of the present invention, performed in a specially designed installation.

### DESCRIPTION OF THE INVENTION

The biogas production method in the methane fermentation process according to present invention, where biomass or waste of vegetable, animal, agricultural or industrial origin is used as a substrate, with the use of bacterial inoculum coming from the primary culture conducted on cattle slurry and fermentation process runs with subsequent stages of hydrolysis, acidogenesis, acetaneogenesis and methanogenesis is based on three stages, each one running in a separate chamber and all the chambers are connected to each other and form a linear system.

Three stages are as follows:
1. In the first stage, the substrate is mixed, homogenized and then the batch is molded, with dry matter content ranging from 10 to 25%. Subsequently, the batch is inoculated with an inoculum of hydrolysis, acidogenesis and acetaneogenesis bacteria just prior to feeding of the acid-hydrogen fermentation chamber. The bacterial inoculum is derived from the post-fermentation process digestate and is subjected to aerobic treatment prior to inoculation. The C:N ratio of the batch formed from substrates is from 14:1 to 40:1. The duration of the first stage is between 90 and 180 minutes. The process is performed in the inoculum and mixing chamber.
2. In the second stage, the batch obtained in the first stage is fed with a pump to the acid-hydrogen fermentation chamber with volumetric flow rate not less than 1,5 m³/min. The process is carried out at a pH of 2.5 to 4.5 and overpressure ranging between 100 and 200 mbar, for 6 to 24 hours.
3. In the third stage, the batch obtained in the second stage, in the form of pulp, is cyclically fed with a pump with volumetric flow rate not less than 1,5 m³/min to the methane fermentation chamber, having a longitudinal shape through which the pulp is moved only in one direction from the beginning of the chamber to its end. The process is performed in pH range 7.2 to 7.5 at the start of the chamber up to pH 7.8 - 8.2 at the end of the chamber. The process overpressure in this stage is between 100 and 200 mbar. The flow time of the pulp portion through the methane fermentation chamber is 20 - 25 days. After this time, the pulp is already fermented and the biogas containing methane is collected from the end of the methane fermentation chamber.

Preferably, in the first stage the batch is formed with dry matter content from 10 to 15%.

Substrates for biogas production may be formulated every day in any proportions, from any biomass and biowaste components, and assuming a proper ratio C:N. Likewise, the batch pH is not important and can be very different depending on the types of the substrates used and may range from pH 2.0 to pH 9.0. The substrate is selected so that the dry matter content is preferably from 10 to 15%. However, for some substrates, the content of dry matter, despite being liquid, is almost 70%. An example of such a substrate that is desirable for biogas production is molasses. The bacterial inoculum obtained from the liquid fraction of its own digestate, received at the end of the fermentation process cycle, is stored and subjected to oxygen treatment. The methaneogenic bacteria are absolute anaerobes and die in contact with oxygen, unlike other strains that are relative anaerobes and can survive under aerobic conditions. The bacterial inoculum used in the process comes from its own primary culture conducted on cattle slurry obtained from the area where the process is being conducted. Moreover, during storage, the digestate temperature is decreasing.

Second and third stage processes are therefore at overpressure between 100 and 200 mbar, which is different than in biogas plants operating in NaWaRo technology and related, working at overpressure between 3 and 5 mbar. The low pH in acid-hydrogen fermentation chamber in the second stage, high overpressure in the second and third stage, and reaction dynamics are obtained by mixing the substrates and adding in the first stage the digestate with inactivated methane bacteria.

Preferably, in the first stage the substrate is acidified, particularly preferably by vinegar, added in amount of 13 to 19 g/dm³ contained in a mixture 3:2 mass of apple pomace mixture and fruit loads containing more than 30% by weight of sugar, previously subjected to acetic fermentation. Adding the substrates and grape vinegar inoculum, already in the first stage to the mixture, in the mixing chamber, significantly influences the further deep acidification of the acid-hydrogen chamber. The addition of bacteria inoculum of hydrolysis, acidogenesis and acetategenesis, and acidification with vinegar, produces about 5 to 28 g/dm³ of acetic acid, which is more than several dozen times higher than in conventional systems.

Acetic acid is then a substrate for the synthesis of methane. Moreover, providing low pH optimal for bacteria of hydrolysis, acidogenesis and acetaneogenesis can shorten the processes taking place in the acid-hydrogen fermentation chamber for one day and even up to 6 hours.

In this way, an intensification of organic matter decomposition processes takes place in the second stage, which results in the extraction of the substrate, which is extremely high in the world-scale. Digestion is at the level of 98.5%.

Preferably, the digestate additive with bacterial inoculum added in the first stage constitutes 8 to 14% of the total volume of the batch.

Preferably, the pulp is loaded into methane fermentation chamber in the amount providing biogas production for a period of 2 to 3 hours.

Adding higher amount would result in a breakdown of alkaline buffer and process distortion. Application of smaller quantity does not allow for the desired bubbling. Favorably, the pulp is fed to methane fermentation chamber in the amount that ensures free gas zone left above the pulp surface.

Preferably, the gas formed in the second stage, containing carbon dioxide and hydrogen, is transported from the acid-hydrogen fermentation chamber to the methane fermentation chamber, directly to its gas zone.

Pumping of the batch with volumetric flow rate, as mentioned into the acid-hydrogen fermentation chamber helps to obtain very strong and violent decomposition processes already taking place within few minutes after batch application. There are intense biological reactions. Mixing and acidification of the charge ensures stable, acidic pH. Similar situation takes place when the pulp is loading to the methane fermentation chamber in the third stage.

Namely, when pumping the portion from the acid-hydrogen chamber into the methane fermentation chamber, a very strong reaction occurs, causing rapid emission of methane and carbon dioxide, connected with increase of overpressure in the methane fermentation chamber even up to 200 mbar in 10 to 20 minutes.

Additionally, supplementation of pulp in this amount, that there is left a free space over its surface in methane fermentation chamber - the gas zone, enables intensification of the biogas production process by partial production in the gas zone. In the gas zone, under given conditions, there is a unique process that enhances the efficiency of the described technology.

There is, as a result of biochemical reactions, the additional synthesis of methane from carbon dioxide and hydrogen, provided by a tubular connection from the second stage of the process, i.e. acid-hydrogen fermentation. The presence of methane bacteria contained in the so-called biological aerosols in the gas zone of the methane fermentation chamber is ensured due to intense supplementation of acidic content from acid-hydrogen fermentation from the second stage to slightly alkaline content of the methane fermentation chamber in the third stage, when very intense reactions of breaking of the buffer capacity occur, combined with immediate, intensive gases emissions. Mainly these are the following gases: CO₂, CH₄ and H₂. The effect of violent bubbling occurs, which in turn causes the peeling of very tiny particles of pulp containing methane bacteria. These gas-floating autotrophic bacteria are responsible for the rapid, lasting less than one second, process of methane molecules formation in the presence of available raw materials i.e. supplemented carbon dioxide and hydrogen.

It has been estimated that even up to 30% of methane is formed in this way in the gas zone. This process is practically non-existent in other fermentation technologies due to the residual fraction of less than 1% of free hydrogen in the evolving biogas, since there is no separated stage of acid-hydrogen fermentation and gaseous overpressure for typical fermentation is several tenths lower. Additionally, the resulting gases are discharged to the outside. It has been investigated that in the described technology the hydrogen content in the gases from acid-hydrogen fermentation chamber may reach even 20-40%.

Preferably, the temperature of the second stage of the process ranges between 30 to 35°C.

The process temperature should not exceed 35°C, as the feeding of the higher temperature pulp to the third stage results in a strong exothermic process that inhibits the methane production as a result of methane bacteria degradation.

Preferably, the pulp is taken from the end part of the methane fermentation chamber and is subjected to irrigation in the gas zone of the final part of the chamber.

Consumption from the final part is a solution that increases methane production by an average 3 - 4%. Due to diluting the droplets of wet pulp, moving through the atmosphere of the carbon dioxide and methane mixture, capture CO₂ which is better soluble in water than CH₄.

Therefore, falling back they increase the availability of CO₂. It is particularly desirable at the end of the chamber, since in this part there is already low content of acetic acid, which has been used to methane synthesis on earlier sections of the methane fermentation chamber. In the end part, there is a large amount of methanogenic autotrophic bacteria capable to direct methane production from CO₂. Concentration of acetic acid close to zero in the final part of the methane fermentation chamber is also a different situation compared to typical fermenters in European biogas plants, because in normal working installations an acetic acid concentrations never drop below 1g/dm³ of the pulp. A typical expert assessing the performance of a biogas plant, seeing the acetic acid content in the fermenting pulp near zero, would say that the fermentation process does not take place. Meanwhile, in the described technology, the lack of acetic acid in the final part of the fermenter proves an optimal running process.

The method of the present invention allows to obtain biogas containing about 56-67% of methane. In addition to the demonstrated efficiency, this method is distinguished by the remarkable dynamics of the processes, resulting from the deep decomposition of the substrates in the acid-hydrogen chamber into the form of readily decomposable straight compounds, finally to acetic acid. After 6 hours of lack of feeding of methane fermentation chamber, biogas production in installation drops by 50%, whereas in typical biogas plant this effect occurs only after few days. On the other hand, when the substrate from the acid-hydrogen chamber is fed to the methane fermentation chamber, the biogas production starts immediately. It means that a very large proportion of the substrates are decomposed to the level of volatile fatty acids, especially acetic acid, so that methanogens can quickly process these acids into methane. Due to this unprecedented dynamics of production growth and rapid stopping, described installation can also be dedicated to work as a so-called peak installation, working in the period of 24 hours when demand for energy is the highest, i.e. from 6:00 am to 8:00 pm.

The method of hydrogen production, carried out by means of biogas production in methane fermentation process, according the biogas production method, is characterized in that the substrate in the inoculum and mixing chamber is homogenized and then used to form a batch with a dry matter content from 10 to 15%. The obtained batch is transported to the acid-hydrogen fermentation chamber and is inoculated with a bacterial inoculum, containing the predominantly methanogenic bacteria, which source is the digestate obtained in the biogas production process, carried out by means of biogas production in methane fermentation process according to the afore-mentioned invention during transportation or in the acid-hydrogen fermentation chamber. Subsequently, obtained gas mixture containing hydrogen is received from the acid-hydrogen fermentation chamber.

Preferably, the additive of digestate with bacterial inoculum, added in the second step to the acid-hydrogen fermentation chamber is from 15 to 35% of the total volume of the batch.

Preferably, vinegar is added to the substrate in order to acidify the substrate in the amount of 13 to 19 g/dm³, in a mixture 3:2 from apple pomace and fruit mix containing more than 30% of sugar, previously subjected to acetic fermentation.

For the production of hydrogen, obtained purely in a mixture with CO₂, without the participation of methane, the addition of methane-rich bacteria with a high degree of concentration, i.e. taken directly from the end of the third stage of fermentation, is used. It is paradoxical, because the addition of methane bacteria resulted in the immediate production of methane, besides hydrogen and carbon dioxide, according to the research conducted under the HYVOLUTION project (019825-SES6) performed under EU's 6th Framework Program. In the discussed technology the methane production process in the second stage does not occur.

Preferably, the pulp obtained in the acid-hydrogen fermentation chamber, upon receipt of hydrogen, is directed to the methane fermentation chamber and biogas is produced therefrom.

Hydrogen is collected after about 6 hours. Subsequently, the pulp is used to feed the methane fermentation chamber in 2-3 hour cycles. The hydrogen production process, according to this invention, produces a gas containing up to 40% hydrogen.

The production process of a chelate-containing mineral fertilizer is characterized by the fact that it is obtained in the biogas production process described above. The process temperature in the second stage ranges between 30 - 35°C.

The low pH maintained in the second fermentation stage and the temperature in the range of 30-35°C are hygenisation factors of the process, because both pathogenic bacteria (*Salmonella* and *E. Coli*) and parasite eggs, such as human or porcine worms, do not survive residence in the acid-hydrogen chamber.

Optionally, when using substrates coming from, for example, a slaughterhouse, prior to application of the substrates to the inoculum and mixing chamber, it is advisable to carry out an initial hygienisation according to known methods.

Preferably the obtained digestate is used to prepare a fertilizer, whereby the digestion degree is up to 98,5%. Fertilizer containing chelate minerals is distinguished by the fact that it is a liquid digestate obtained during the production of the biogas, according to the present invention, and the degree of its digestion is up to 98.5%.

The digestate achieved by the described biomass processing technology is characterized by unique features that determine the following product functions:
- fertilization of plants is more effective,
- plants are protected from pathogens,
- increased resistance of plants to difficult weather conditions such as resistance to drought, wintering/hibernation.

The specific properties of fertilizers and biological properties of digestate are determined by:
- high content of potassium and iron,
- nitrogen in the form of ammonia,
- high availability of organic chelating compounds,
- specific structure of the content of particular organic acids.

The function of effective fertilization is related to high potassium content, which directly influences plant yield through photosynthesis, activates enzymes and plays a role in nitrogen economy/management. In turn, the presence of iron in the digestate, especially in chelated form, is important for optimizing the production of chlorophyll in the plant, which directly affects plant development and yield. The high efficiency of plant support with fertilizers is determined by the deep hydrolysis of the substrates during the process.

This, in turn, determines the high degree of digestate digestion, reaching 98.5%. Due to such high digestion, high availability of organic chelating compounds in the digestate such as amino acid and micro- and macroelements in the form of easily absorbed chelates has been achieved. The fact that the ammonium form (N-NH₄) is a significant part of the total nitrogen content also plays a significant role in the fertilizer value of the digestate. Ammonium form is easily absorbed by the plants, both in foliar and in soil applications.

The functions of plant protection against pathogens, mainly fungal diseases, described digestate, are related to high potassium content, relatively high sodium content and organic acid composition, resulting from fermentation processes intensities, with propionic and caproic acid predominance.

### Detailed description

The invention in the exemplary implementations is shown below.

For better understanding, the methods according to inventions are carried out, without limitation in a biogas installation, shown on drawings, where:
Fig. 1 schematically illustrates an installation plan for biogas and hydrogen production.
Fig. 2 shows enlarged "A" fragment, marked in Fig.1
Fig. 3 shows the methane fermentation chamber in A-A cross-section, shown in Fig.2

The aforementioned installation serves only to highlight technical aspect of the claimed methods and is not a part of the present invention.

### Exemplary biogas and hydrogen installation

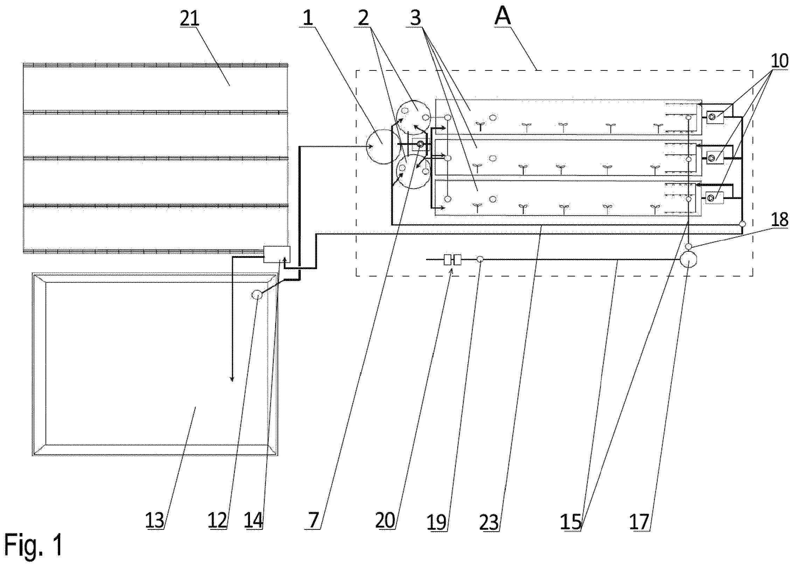

The biogas installation shown in Fig. 1, Fig. 2 and Fig. 3 simultaneously serves to produce hydrogen. The installation comprises inoculum and mixing chamber 1, which is connected in this case by a channel with two hydrogen-acid fermentation chambers 2, and in turn these chambers are connected with three methane fermentation chambers 3. Chambers 1, 2 and 3 are made of reinforced concrete. Hydrogen-acid fermentation chambers 2 and methane fermentation chambers 3 are closed sealed tanks designed for overpressure not less than 250 mbar produced in their interior. Chambers 1 and 2 in the present performance have a circular cross-section. Furthermore, chambers 1 and 2 are equipped with mixing stirrers converting the substrate. Methane fermentation chambers 3 have a longitudinal shape and a circular cross section. The length of chambers 3 is 90m. The length and the longitudinal shape let the pulp pumped into the methane fermentation chamber 3 slowly move in its interior from the start to the end part, and is progressively subjected to changing methane fermentation processes. Chambers 3 have a biogas intake 6. The pipes transporting the substrate from the first stage to the second one and the pulp from the second stage to the third stage are carried out by the first pumping station 7. The first pumping station 7 is equipped with a pump set not shown in the drawing, that allows the material to be forwarded with the desired dynamics. Regulation of the flow in appropriate directions is achieved by means of suitable sliders. On the schematics, the direction of adequate material handling, at given stage, is indicated by arrows.

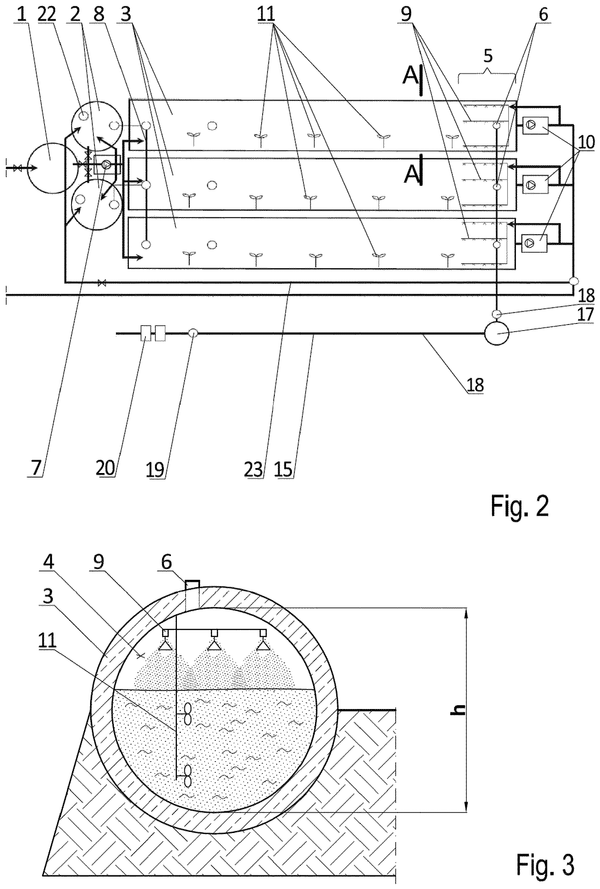

In each methane fermentation chamber 3 is the gas zone 4, as shown in Figure 3. The gas zone 4 is the free space formed over the surface of the pulp moving along the chamber 3.

Into the gas zone 4, there is introduced a gaseous pipe 8 from the acid-hydrogen fermentation chamber 2, which supplies obtained in chamber 2 gases: hydrogen and carbon dioxide, which are then used in the methane fermentation chamber 3 for methane synthesis and have a significant effect on the process yield.

In the end part 5 of each methane fermentation chamber 3, in the gas zone 4 there is a pulp spreading system 9 supplied from the second pumping station 10 receiving the digestate. The end part 5 has no strictly defined length, and the location of the pulp spreading system 9 in this part results from the fact that the dampened wet droplets of the pulp move through the atmosphere of the carbon dioxide and methane mixture, capturing CO₂ that is better soluble in water than CH₄. And therefore, falling back they increase the availability of CO₂.

At the end of chamber 3 is already low content of acetic acid, which was used to synthesize methane on earlier sections of methane fermentation chamber 3. At the end of section 5, however, there is a significant amount of methaneogenic autotrophic bacteria capable to produce methane directly from COz, which helps to digest non-fermented substrate molecules and thereby, boosts methane production. Methane fermentation chamber 3 is equipped on its length with the stirrers 11 to prevent the formation of bottom sediments and a layer of coat cover on the surface of the sliding pulp. The stirrers do not cause the pulp movements towards the end of the chamber 3. The installation has a station 12, deactivating methanogenic bacteria, equipped with a stirrer. In this performance, station 12 is connected to inoculum and mixing chamber 1. Station 12 takes the digestate from the lagoon 13, where it is pumped from the end of methane fermentation chamber 3 through the second pumping station 10. Prior to feeding the lagoon 13 the digestate is sent to the separator 14. From the 5 biogas intake, the obtained biogas gets with line 15 to the desulphuriser 17, after reducing its overpressure to about 130 mbar through the reducer 18. Subsequently, the biogas is directed to the condensate well from biogas 19 and further to the dehydration and drainage station 20. The installation is also equipped with the silos 21 for silage. Specification of particular stages and their performing in separate chambers creates ideal conditions for the development of particular microbial groups with different optimal pH levels. The inoculum and mixing chamber 1 enables the substrate to be inoculated with a suitable bacterial inoculum and batch formation. In a separated, closed acid-hydrogen fermentation chamber 2, a second stage is followed, where the hydrolysis, acidogenesis and acetanogenesis phases occur. Whereas, in the methane fermentation chamber 3, an appropriate third stage, is realized in the methanogenesis process.

For hydrogen production, the hydrogen-acid fermentation chamber 2 has a hydrogen capture 22 and the hydrogen-acid fermentation chamber 2 is directly connected to the second pumping station 10. This connection is carried out by the line 23. It enables the delivery of digestate containing bacterial inoculum under anaerobic conditions necessary for survival of methanogenic bacteria. The second pumping station 10 thus pumps the digestate received from the end of the methane fermentation chamber 3 to the pulp spreading system 9, to the separator 14 and to the lagoon 13, as well as pumps the digestate with inoculum containing methanogenic bacteria into the hydrogen-acid fermentation chamber 2 in order to produce hydrogen.

The biogas installation, as described above, may also serve to conduct hydrogen production. A direct connection of the acid-hydrogen fermentation chamber 2 to the second pumping station enables the substrate to be inoculated with methanogen bacteria, which are absolute anaerobes. It is obvious that hydrogen production could take place independently of biogas production and in an installation limited only to the inoculum and mixing chamber 1 as well as acid-hydrogen chamber 2. However, it would be a requirement to provide the bacterial inoculum obtained from the fermentation chamber 3 in biogas production. Additionally, the pulp obtained after hydrogen production would have to be disposed, which would lead to wastage of still valuable biogas material, and moreover the recycling would be a significant problem. Therefore, it is economically unreasonable and troublesome to produce hydrogen independently of biogas production. Therefore, it is particularly advantageous to produce hydrogen in the same installation as biogas and, for example, alternately, or depending on current demand.

### Example 1.

The sludge from the rapeseed industry was used for biogas production. The process was carried out in the biogas and hydrogen generation installation shown in the attached drawings. For the processing into biogas of 10 Mg of rapeseed sludge from rapeseed presses containing 58% of dry matter and 94% of dry organic matter, this waste material was mixed in the first stage with 10 Mg of maize silage with content of 35% of dry matter and 52 Mg treacle distillery with 7% of dry matter. The substrate with a dry matter content of 12.9% and a carbon - nitrogen ratio of C:N = 40:1 was obtained. The substrate was mixed and homogenized in the inoculum and mixing chamber 1 shown in Fig. 1 and Fig. 2, within 3 hours. In order to force high dynamics of fermentation processes taking place in the acid-hydrogen fermentation chamber, 5 Mg of the vinegar mixture was added to the substrate. The mixture was obtained from 3 Mg apple pomace and 2 Mg fruit loads with sugar content of more than 30% by mass. The mixture was previously subjected to acetic fermentation. From this substrate a vinegar containing acetic acid was formed. Liquid digestate was added at 12% of the total volume of the batch. The digestate contained a bacterial inoculum and was obtained in the biogas production process in previous cycles. Digestate, before being added to inoculum and mixing chamber 1, was subjected to aerobic treatment by aerating in order to eliminate methanogen bacteria. The inoculum included bacteria of hydrolysis, acidogenesis and acetaneogenesis.

After mixing, intended for homogenization for pumpability, the substrate was ready for use in the second stage. The substrate was fully pumped with a high efficiency of 100 m³ / h into the acid-hydrogen fermentation chamber 2 in an hour. In the acid-hydrogen fermentation chamber 2 there were already 40 m³ of liquid inoculum from the previous strain containing the hydrolysis, acidogenesis and acetaneogenesis bacteria. In acid-hydrogen fermentation chamber 2, at 35°C temperature, three phases of fermentation: hydrolysis, acidogenesis and acetateogenesis, occured intensely. After 6 hours, very high saturated acetic acid level of 6000 mg CH₃COOH / liter of bath was obtained. The acid content at this level and consequently the low pH and the added bacterial inoculum, for which the pH value of 2.5-3.5 gives optimum living and development conditions, enabled the dynamic reaction to produce overpressure of 150 mbar. The batch prepared in acid-hydrogen fermentation chamber 2, currently in the form of pulp, was cyclically forwarded every 2 hours to methane fermentation chamber 3 where third stage was running. As before, the pulp was pumped into the methane fermentation chamber 3 with high efficiency of 100 m³/h. The elongated shape of the chamber 3 allowed the pulp to move only in one direction from the beginning of the chamber 3 to its end. The process was performed in pH range of approximately 7.3 at the beginning of chamber 3 to pH value 8.0 at the end. At this stage, with overpressure of 150 mbar, there was an intense methanogenesis process. Gas overpressure measurements showed a very dynamic increase in biogas production - within the first few minutes from the start adding the substrate from the acid chamber. Feeding the pulp with indicated dynamics and in a certain amount resulted in the filling of the chamber 3 to a height of 0.6 h of its height in the light. It enabled to create in the methane fermentation chamber 3, above the pulp surface, the gas zone 4. Gas containing carbon dioxide and hydrogen, created in the second stage in acid-hydrogen fermentation chamber 2 was transferred to the gas zone 4. Due to this, it was possible in the gas zone 4 to intensify the formation of methane particles, as the intensively charged acidic pulp reacted violently with the basic content of the chamber 3, derived from the previous cycle of the process. The violent bubbling caused the peeling of very fine particles of methane-containing pulp, which, by creating biological aerosols, produced a rapid, under 1 second, methane particle formation process in the presence of added carbon dioxide and hydrogen.

Furthermore, in the third stage, the pulp was also taken from the final part 5 of the methane fermentation chamber 3 and subjected to smeared in the gas zone 4, of the end part 5 of the chamber 3. The duration of the third stage in which the entire portion of the pulp passed through the chamber 3 and was digested amounted 22 days. After this time, the pulp portion was digested in 95% and methane-containing biogas was collected from the methane fermentation chamber 3. As a result of the process, 10400 m³ of biogas containing 60% methane were obtained and 25.3 MWh of electricity was produced.

### Example 2

Biogas production was carried out using substrates with predominance of the distillery, vegetable, dairy and maize silage and sludge waste materials.

The process was performed in the installation for biogas and hydrogen production, shown in the attached drawing. A monthly survey of operating efficiency of 1.2 MW biogas plant was conducted.

Delivery of the abovementioned substrates to the installation was done by feeding the waste in equal, fresh mass doses in 2 to 2.5 hour interval for the entire 24-hour period, including statutory holidays. On average, approximately 9.8 Mg of substrate was fed in a single month, taking into account 12% by volume of digestate additive as bacterial inoculum accelerating fermentation process, free of methanogenic bacteria, in analogy to example 1.

The following amount of waste used as a biogas batch was delivered during the month:

| Substrate | Fresh mass [Mg] | Dry mass [Mg] |
|---|---|---|
| Maize silage | 183 | 61,3 |
| Distillery | 1801 | 135,16 |
| Whey permeat | 572 | 125,64 |
| Apple pomaces | 350 | 84,06 |
| Waste vegetables and fruits | 5,58 | 0,56 |
| Expired yogurts | 20,98 | 3,15 |
| Cake | 6,86 | 1,37 |
| Expired frozen vegetables | 24,06 | 2,41 |
| Sludge from the sewage treatment plants | 264,31 | 69,62 |

In total, 3227.79 Mg of fresh mass of substrates was used, containing 483.27 Mg of dry matter. The C:N ratio of the batch was in the range of 14: 1 to 40: 1. The process was carried out analogously to Example 1, except that the time of pulp flow was 25 days. In the test month, a total of 769 MWh of electricity was produced. It was achieved due to production of 374674 m³ of biogas containing 215438 m³ of methane in the fermentation process. As a result, biogas production was 116.08 m³ from 1 Mg fresh substrate and 775.29 m³ from 1 Mg dry matter.

In turn, the efficiency of methane production was 66.74 m³ from 1 Mg fresh weight and 445.79 m³ from 1 Mg dry matter. The efficiency of biogas production from 1 Mg dry matter, at this composition of substrates, with the dominance of distillery, vegetable, dairy and maize silage and sludge, was higher by about 150-200 m³, yield of 1 Mg dry matter of mixture of plant and waste substrates obtained in traditional and most widespread in Europe NaWaRo technology.

As a result of the decomposition of organic-mineral complexes in the process of hydrolysis and acid- and acetanogenesis in the second stage, micronutrients were released which, in the ready-to-use digestate, are directly accessible to plants and microorganisms in the form of biochelates. The obtained digestate is liquid and acts as ready-made plant fertilizer and antifungal plant protection agent.

The obtained digestate in the analyzed example possessed very high fertilization properties, which consisted of:
- nitrogen in NH₄ ammonium form: 0.1% in fresh mass of the resulting liquid digestate;
- phosphorus P: - 0.99% in dry mass (d.m.) of obtained liquid digestate;
- potassium K: 13,6% in d.m.;
- magnesium Mg: 0.35% in d.m.;
- sodium Na: 2.26% in d.m.;
- calcium Ca: 2.21% in d.m.;
- sulfur S: 0.83% in d.m.;
- copper Cu: 20 mg / kg in d.m.;
- nickel Ni: 9.6 mg / kg in d.m.;
- zinc Zn: 173 mg / kg in d.m.;
- iron Fe: 5050 mg / kg in d.m.;
- beryl Be: 32 mg / kg in d.m.;
- cobalt Co: 1.24 mg / kg in d.m.;
- manganese Mn: 171 mg / kg in d.m.;
- molybdenum Mo: 1.9 mg / kg in d.m.;
- selenium Se: 0.38 mg / kg in d.m.;

### Example 3.

The substrates dominated by dairy, distillery and sugar waste were used in hydrogen and biogas production. The process was performed in the installation for biogas and hydrogen production shown in the attached drawing. A 4-day study of the operating efficiency of a 1.2 MW biogas plant was conducted. The purpose of the tests was to determine the intensity of hydrogen production from acid-hydrogen chamber 2 and biogas from methane ferment chamber 3.

The settings of the acid-hydrogen fermentation chamber 2 were carried out by providing the substrates mixture once a day.

During the study, the following amount of waste was used as input to the biogas plant:

| Substrate | Fresh mass [Mg] | Dry mass [Mg] |
|---|---|---|
| Pressed beet pulp | 12,60 | 2,55 |
| Distillery | 64,00 | 4,61 |
| Whey permeat | 161,60 | 33,09 |
| Apple pomaces | 19,20 | 4,61 |
| Expired frozen vegetables | 2,49 | 0,25 |
| Sludge from the sewage treatment plants | 45,24 | 9,07 |

During the research on biogas plant functioning on the abovementioned substrates a total of 305.13 Mg fresh weight was used, which gives 54.18 Mg dry matter. On average it gives 76.28 Mg fresh and 13.55 Mg dry matter per day. The batch with given composition, after mixing and homogenizing, was fed in this amount once every 24 hours to the acid-hydrogen chamber 2. In chamber 2, the digestate of 12% of the total volume of the substrate was added. The digestate contained a bacterial inoculum and was obtained in the biogas production process in previous cycles.

The inoculum was not oxidized as in Example 1 or 2, so methanogenic bacteria were predominated. The batch was further acidified in the inoculum and mixing chamber 1 by adding 5 Mg of vinegar mixture, obtained from 3 Mg apple pulp and 2 Mg of fruit pulp with a sugar content higher than 30% by mass. The mixture was previously subjected to acetic fermentation. Vinegar containing acetic acid was formed in this mixture.

Gas produced in the acid-hydrogen fermentation chamber 2 contained about 39% H₂ concentration and about 41% CO₂ concentration. Daily gas production amounted 1540 m³ during testing. It was observed that, after batch and of methanogenic inoculum application, an intense hydrogen production lasted about 6 hours. The study tests were conducted in a series of four repetitions, in total 4 days of the study cycle. The pulp obtained after the hydrogen production process was directed to the methane fermentation chamber 3 and further biogas production was carried out under the same conditions as in Example 1.

## Claims

1. Method for biogas production in methane fermentation process, wherein biomass or waste of vegetable, animal, agricultural or industrial origin is used as the substrate, with the use of bacterial inoculum coming from the primary culture conducted on cattle slurry and the fermentation process is performed including the phases of hydrolysis, acidogenesis, acetaneogenesis and methanogenesis, **characterized in that** it is carried out in three stages, where every single stage runs in a separate chamber, and these chambers are connected to each other and form a linear system and furthermore:
- in the first stage, the substrate is mixed, homogenised and therefrom the batch is formed with dry matter content from 10 to 25%, to which subsequently an inoculum of hydrolysis, acidogenesis and acetaneogenesis bacteria is introduced, wherein the bacterial inoculum is derived from the obtained digestate and before inoculation is subjected to aerobic treatment, Z the C:N ratio of the batch formed from substrates is from 14:1 to 40:1, and the duration of the first stage is 90 to 180 minutes and the process is performed in the inoculum and mixing chamber (1);
- in the second stage, the batch obtained in the first stage is fed with a pump with volumetric flow rate not less than 1,5 m³/min to the acid-hydrogen fermentation chamber (2) and the process is carried out at pH value 2.5 to 4.5 and overpressure between 100 and 200 mbar for 6 to 24 hours;
- in the third stage, the batch obtained in the second stage, in the form of pulp, is fed cyclically with a pump with volumetric flow rate not less than 1,5 m³/min to the methane fermentation chamber (3), having a longitudinal shape through which the pulp is moved only in one direction from the beginning of the chamber (3) to its end, the process is carried out in pH range between 7.2 to 7.5 at the start of the chamber (3) to pH 7.8 to 8.2 at the end of the chamber (3) and overpressure between 100 and 200 mbar, time of pulp portions flow through the methane fermentation chamber is 20 to 25 days and after that from the methane fermentation chamber (3) the biogas containing methane is collected.

2. Method according to claim 1, **characterized in that** in the first stage the batch is formed with dry matter content from 10 to 15%.

3. Method according to claim 1 or 2, **characterized in that** the first stage resulting substrate is acidified.

4. Method according to claim 3, **characterized in that** the substrate is acidified with vinegar, added in the amount of 13-19 g/dm³, contained in a mixture 3:2 apple pomace and fruit pulp with sugar content over 30%, previously subjected to acetic fermentation.

5. Method according to any of the preceding claims, **characterized in that** the additive of the digestate with bacterial inoculum added in the first stage to the inoculum and mixing chamber (1) is from 8 to 14% of the total volume of the batch.

6. Method according to any of the preceding claims, **characterized in that** the pulp is fed to methane fermentation chamber (3) in the amount that ensures biogas production for a period of 2-3 hours.

7. Method according to any of the preceding claims, **characterized in that** the pulp is fed to methane fermentation chamber (3) in the amount that ensures leaving the free gas zone (4) above the pulp surface.

8. Method according to any of the preceding claims, **characterized in that** the gas formed in the second stage, containing carbon dioxide and hydrogen, is transported from the acid-hydrogen fermentation chamber (2) to the methane fermentation chamber (3), directly into its gas zone (4).

9. Method according to any of the preceding claims, **characterized in that** temperature of the process in the second stage is between 30 and 35°C.

10. Method according to any of the preceding claims, **characterized in that** in the third stage the pulp is taken from the end part (5) of methane fermentation chamber (3) and is subjected to diffusion in the gas zone (4) at the end part (5) of the chamber (3).

11. Method for hydrogen production, carried out by means of biogas production in methane fermentation process, according to the claims 1-10, **characterized in that** the substrate in the inoculum and mixing chamber (1) is homogenized and therefrom a batch is formed with dry matter content 10 - 15% and C:N ratio between 14:1 to 40:1 and subsequently obtained batch is forwarded to acid-hydrogen fermentation chamber (2) and during transportation or in the acid-hydrogen fermentation chamber (2) bacterial inoculum is introduced, containing predominantly methanogenic bacteria, which source is - the digestate obtained in the biogas production process according to the claims 1-10; and then resulting gas mixture containing hydrogen is received from acid-hydrogen fermentation chamber (2).

12. Method according to claim 11, **characterized in that** addition of digestate with bacterial inoculum comprises 15 - 35% of the total volume of the batch.

13. Method according to claims 11 - 12, **characterized in that** the substrate is acidified in inoculum and mixing chamber (1).

14. Method according to claim 13, **characterized in that** the substrate is acidified with vinegar added in amount 13 - 19 g/dm³, contained in a mixture 3:2 of apple pomace and fruits pulp containing sugar more than 30%, previously subjected to acetic fermentation.

15. Method according to claims 11-14, **characterized in that** the pulp obtained in the acid-hydrogen fermentation chamber (2) upon receipt of hydrogen is directed to the methane fermentation chamber (3) and biogas is produced therefrom.

16. Method according to claims 1-10 further comprising preparing a fertilizer containing mineral compounds in the form of chelates, **characterized in that** the process temperature in the second stage ranges between 30 to 35°C.

17. Method according to claim 16, **characterized in that** it is a liquid digestate obtained by the method of claim 16 with a digestion degree of up to 98,5%.

## Patentansprüche

1. Verfahren für Herstellung von Biogas im Prozess der Methangärung, bei dem Biomasse oder Abfälle pflanzlichen, tierischen, landwirtschaftlichen oder industriellen Ursprungs als Substrat verwendet werden, unter Verwendung von bakteriellem Inokulum aus einer auf Rindergülle durchgeführten Primärkultur, wobei ein Gärungsprozess durchgeführt wird, der Phasen der Hydrolyse, Säurebildung, Acetatbildung und Methanbildung umfasst,
**dadurch gekennzeichnet, dass**
es in drei Phasen durchgeführt wird, wobei jede Phase in einer separaten Kammer verläuft und diese Kammern miteinander verbunden sind und ein lineares System bilden, ferner:
- In der ersten Phase wird das Substrat gemischt, homogenisiert und zu einer Charge mit einem Trockensubstanzgehalt von 10 bis 25 % geformt, in das dann das bakterielle Inokulum aus Hydrolyse, Säurebildung und Acetatbildung eingebracht wird, wobei das bakterielle Inokulum aus dem erhaltenen Gärungsprodukt stammt und vor dem Saat einer aeroben Behandlung unterzogen wird und das C:N-Verhältnis der aus den Substraten gebildeten Charge 14:1 bis 40:1 beträgt und die Dauer der ersten Phase 90 bis 180 Minuten beträgt und der Prozess im Inokulum und in der Mischkammer (1) stattfindet;
- In der zweiten Phase wird die in der ersten Phase gewonnene Charge mittels einer Pumpe mit einem Volumenstrom von mindestens 1,5 m³/min der Sauer-Wasserstoff-Gärkammer (2) zugeführt und der Prozess wird bei einem pH-Wert von 2,5 bis 4,5 und einem Überdruck von 100 bis 200 mbar 6 bis 24 Stunden lang durchgeführt;
- In der dritten Phase wird die in der zweiten Phase gebildete Charge mittels einer Pumpe mit einem Volumenstrom von mindestens 1,5 m³/min einer Methangärungskammer (3) zugeführt, die eine längliche Form aufweist, in welcher die Pulpe in nur einer Richtung sich bewegt und zwar vom Anfang der Kammer (3) zu deren Ende und der Prozess in einem pH-Bereich von 7,2 bis 7,5 am Anfang der Kammer (3) bis pH von 7,8 bis 8.2 am Ende der Kammer (3), bei einem Überdruck von 100 bis 200 mbar, wobei dir Durchflusszeit einer Pulpenportion durch die Methangärungskammer 20 bis 25 Tage beträgt und dann von der Methangärungskammer (3) methanhaltiges Biogas entnommen wird.

2. Verfahren nach Anspruch 1, **dadurch gekennzeichnet, dass** in der ersten Phase die Charge mit einem Trockensubstanzgehalt von 10 bis 15% gebildet wird.

3. Verfahren nach Anspruch 1 oder 2, **dadurch gekennzeichnet, dass** das in der ersten Phase entstandene Substrat sauer gemacht wird.

4. Verfahren nach Anspruch 3, **dadurch gekennzeichnet, dass** das Substrat mittels Essig in einer Menge von 13-19 g/dm³ sauer gemacht wird, der in einem 3:2-Gemisch von Apfelpressrückständen und Obstpulpe mit einem Zuckergehalt von mehr als 30% enthalten ist, das vorher einer Essiggärung unterzogen wurde.

5. Verfahren nach einem der vorangehenden Ansprüche, **dadurch gekennzeichnet, dass** die Menge des Gärungsproduktes mit dem bakteriellen Inokulum, das in der ersten Phase dem Inokulum und der Mischkammer (1) 8 bis 14% der Gesamtvolumens der Charge beträgt.

6. Verfahren nach einem der vorangehenden Ansprüche, **dadurch gekennzeichnet, dass** die Pulpe der Methangärungskammer (3) in einer Menge zugeführt, welche die Biogasproduktion für die Dauer von 2-3 Stunden gewährleistet.

7. Verfahren nach einem der vorangehenden Ansprüche, **dadurch gekennzeichnet, dass** die Pulpe der Methangärungskammer (3) in einer Menge zugeführt, welche die Bildung einer freien Gaszone (4) oberhalb der Oberfläche der Pulpe gewährleistet.

8. Verfahren nach einem der vorangehenden Ansprüche, **dadurch gekennzeichnet, dass** das in der zweite Phase gebildete Gas, das Kohlendioxid und Wasserstoff enthält, von der Säure-Wasserstoff-Gärungskammer (2) in die Methangärungskammer (3), direkt zu derer Gaszone (4) transportiert wird.

9. Verfahren nach einem der vorangehenden Ansprüche, **dadurch gekennzeichnet, dass** die Prozesstemperatur der zweiten Phase 30 bis 35 °C beträgt.

10. Verfahren nach einem der vorangehenden Ansprüche, **dadurch gekennzeichnet, dass** in der dritten Phase die Pulpe vom Endbereich (5) der Methangärungskammer (3) entnommen und einer Diffusion in der Gaszone (4) am Endbereich (5) der Kammer (3) unterzogen wird.

11. Verfahren für Wasserstoffproduktion durch Produktion von Biogas im Prozess der Methangärung nach Ansprüchen 1-1-, **dadurch gekennzeichnet, dass** das Substrat im Inokulum und in der Moschkammer (1) homogenisiert wird und daraus die Charge mit einem Trockensubstanzgehalt von 10-15 % und C:N-Verhältnis von 14:1 bis 40:1 gebildet wird, wobei anschließend die gebildete Charge der Säure-Wasserstoff-Gärungskammer (2) zugeführt wird und beim Transport oder in der Säure-Wasserstoff-Gärungskammer (2) das bakterielle Inokulum beigemischt wird, das hauptsächlich methanogene Bakterien enthält und vom Nachgärungsprodukt des Biogasproduktionsprozesses stammt, nach Ansprüchen 1-10, wobei dann das wasserstoffhaltige Gasgemisch der Säure-Wasserstoff-Gärungskammer (2) entnommen wird.

12. Verfahren nach Anspruch 11, **dadurch gekennzeichnet, dass** der Zusatz des Nachgärungsproduktes vom bakteriellen Inokulum 15-35% des Gesamtvolumens der Charge entspricht.

13. Verfahren nach Anspruch 11-12, **dadurch gekennzeichnet, dass** das Substrat im Inokulum und in der Mischkammer (1) sauer gemacht wird.

14. Verfahren nach Anspruch 13, **dadurch gekennzeichnet, dass** das Substrat mittels Essig in einer Menge von 13-19 g/dm³ sauer gemacht wird, der in einem 3:2-Gemisch von Apfelpressrückständen und Obstpulpe mit einem Zuckergehalt von mehr als 30% enthalten ist, das vorher einer Essiggärung unterzogen wurde.

15. Verfahren nach Anspruch 11-14, **dadurch gekennzeichnet, dass** die in der Säure-Wasserstoff-Gärungskammer gebildete Pulpe (2) nach der Wasserstoffaufnahme der Methangärungskammer (3) zugeführt wird und daraus Biogas produziert wird.

16. Verfahren nach Ansprüchen 1-10, umfassend ferner die Produktion des Düngers mit Mineralverbindungen in Form von Chelaten, **dadurch gekennzeichnet, dass** die Prozesstemperatur der zweiten Phase 30 bis 35 °C beträgt.

17. Verfahren nach Anspruch 16, **dadurch gekennzeichnet, dass** es sich um ein flüssiges Gärungsprodukt handelt, das nach Anspruch 16 produziert wird und eine Aufschlussrate bis 98,5% aufweist.

## Revendications

1. Un procédé de production de biogaz par un processus de méthanisation dans lequel une biomasse ou des déchets d'origine végétale, animale, agricole ou industrielle sont utilisés comme substrat, avec l'utilisation d'un inoculum bactérien issu d'une culture primaire menée sur du lisier de bovin, et un processus de fermentation comprenant les phases d'hydrolyse, d'acidogénèse, d'acétogénèse et de méthanogénèse est réalisé,
**caractérisé en ce qu'**
il est réalisé en trois étapes, chaque étape se déroulant dans une chambre séparée et les chambres étant interconnectées pour former un système linéaire, et en outre :
- dans la première étape, le substrat est mélangé, homogénéisé pour en former une charge de matière sèche de 10 à 25 %, dans laquelle un inoculum bactérien issu de l'hydrolyse, de l'acidogénèse et de l'acétogénèse est ensuite introduit, l'inoculum bactérien étant issu du digestat obtenu et soumis à un traitement aérobie avant l'ensemencement, et le rapport C:N de charge formée à partir des substrats varie de 14:1 à 40:1, et la durée de la première étape varie de 90 à 180 minutes, le processus se déroulant dans l'inoculum et dans la chambre de mélange (1) ;
- dans la deuxième étape, la charge obtenue dans la première étape est alimentée dans le digesteur acide-hydrogène (2) par l'intermédiaire d'une pompe avec un débit volumétrique non inférieur à 1,5m³/min et le processus est réalisé à un pH de 2,5 à 4,5 et à une surpression de 100 à 200 mbar pendant une période de 6 à 24 heures ;
- dans la troisième étape, la charge obtenue dans la deuxième étape sous la forme de pulpe est alimentée de manière cyclique au moyen d'une pompe avec un débit volumétrique non inférieur à 1,5m³/min dans le digesteur de méthanisation (3), ayant une forme allongée, dans lequel la pulpe se déplace dans une seule direction du début du digesteur (3) à son extrémité, et le processus est réalisé à un pH allant de 7,2 à 7,5 au début du digesteur (3) à un pH allant de 7,8 à 8,2 à l'extrémité du digesteur (3) et une surpression de 100 à 200 mbar, le temps d'écoulement d'une portion de pulpe à travers le digesteur de méthanisation étant de 20 à 25 jours, et du biogaz contenant du méthane est ensuite extrait du digesteur de méthanisation (3).

2. Le procédé selon la revendication 1, **caractérisé en ce que** dans la première étape, la charge avec une teneur en matière sèche de 10 à 15 % est formée.

3. Le procédé selon les revendications 1 ou 2, **caractérisé en ce que** le substrat obtenu dans la première étape est acidifié.

4. Le procédé selon la revendication 3, **caractérisé en ce que** le substrat est acidifié avec du vinaigre, ajouté à 13-19 g/dm³, contenu dans le mélange 3:2 de marc de pomme et de pulpe de fruit contenant plus de 30 % de sucre, préalablement soumis à une fermentation acétique.

5. Le procédé selon l'une quelconque des revendications précédentes, **caractérisé en ce que** l'addition du digestat avec l'inoculum bactérien ajouté dans la première étape à l'inoculum et la chambre de mélange (1) est de 8 à 14 % du volume total de la charge.

6. Le procédé selon l'une quelconque des revendications précédentes, **caractérisé en ce que** la pulpe est alimentée dans le digesteur de méthanisation (3) en une quantité assurant la production de biogaz pendant une période de 2 à 3 heures.

7. Le procédé selon l'une quelconque des revendications précédentes, **caractérisé en ce que** la pulpe est alimentée dans le digesteur de méthanisation (3) en une quantité assurant qu'une zone de gaz libre (4) est laissée au-dessus de la surface de la pulpe.

8. Le procédé selon l'une quelconque des revendications précédentes, **caractérisé en ce que** le gaz formé dans la deuxième étape, contenant du dioxyde de carbone et de l'hydrogène, est transporté du digesteur acide-hydrogène (2) au digesteur de méthanisation (3), directement dans sa zone de gaz (4).

9. Le procédé selon l'une quelconque des revendications précédentes, **caractérisé en ce que** la température du processus dans la deuxième étape est comprise entre 30 et 35 °C.

10. Le procédé selon l'une quelconque des revendications précédentes, **caractérisé en ce que** dans la troisième étape, la pulpe est prélevée de la partie d'extrémité (5) du digesteur de méthanisation (3) et est soumise à une diffusion dans la zone de gaz (4) au niveau de la partie d'extrémité (5) du digesteur (3).

11. Le procédé de production d'hydrogène, réalisé par la production de biogaz dans le processus de méthanisation, selon les revendications 1 à 10, **caractérisé en ce que** le substrat dans l'inoculum et la chambre de mélange (1) est homogénéisé et qu'à partir de celui-ci, la charge avec une teneur en matière sèche de 10 à 15 % et un rapport C:N de 14:1 à 40:1 est formée, puis la charge obtenue est dirigée vers le digesteur acide-hydrogène (2) et, pendant le transport ou dans le digesteur acide-hydrogène (2), l'inoculum bactérien contenant principalement des bactéries méthanogènes est introduit, dont la source est le digestat obtenu dans le procédé de production de biogaz selon les revendications 1-10, puis le mélange gazeux contenant de l'hydrogène résultant est extrait du digesteur acide-hydrogène (2).

12. Le procédé selon la revendication 11, **caractérisé en ce que** l'addition du digestat avec l'inoculum bactérien représente 15 à 35 % du volume total de la charge.

13. Le procédé selon les revendications 11-12, **caractérisé en ce que** le substrat est acidifié dans l'inoculum et la chambre de mélange (1).

14. Le procédé selon la revendication 13, **caractérisé en ce que** le substrat est acidifié avec du vinaigre ajouté à 13-19g/dm³, contenu dans un mélange 3:2 de marc de pomme et de pulpe de fruit contenant plus de 30 % de sucre, préalablement soumis à une fermentation acétique.

15. Le procédé selon les revendications 11-14, **caractérisé en ce que** la pulpe obtenue dans le digesteur acide-hydrogène (2) après absorption d'hydrogène est dirigée vers le digesteur de méthanisation (3) et du biogaz est produit à partir de celui-ci.

16. Le procédé selon les revendications 1-10, comprenant en outre la production d'un engrais comprenant des composés minéraux sous la forme de chélates, **caractérisé en ce que** la température du processus dans la deuxième étape est de 30 à 35 °C.

17. Le procédé selon la revendication 16, **caractérisé en ce qu'**il s'agit d'un digestat liquide obtenu par le procédé selon la revendication 16 avec un taux de dilution allant jusqu'à 98,5 %
